# EUROPEAN PATENT SPECIFICATION

(11) **EP 0 586 002 B1**
(45) Date of publication and mention of the grant of the patent: **19.01.2000**
(21) Application number: 93202428.4
(22) Date of filing: 18.08.1993
(51) Int. Cl.: A61K 39/395, C12P 21/08

(54) **Monoclonal antibodies recognizing the epidermal growth factor receptor, cells and methods for their production and compositions containing them**
Monoklonale Antikörper gegen den epidermalen Wachstumsfaktorrezeptor, Zellen und Verfahren zur ihrer Herstellung und sie erhaltende Zusammensetzungen
Anticorps monoclonaux contre le récepteur du facteur épidermique de croissance, cellules et méthodes pour leur préparation et compositions qui les contiennent

(30) Priority: 18.08.1992 CU 10092; 01.03.1993 CU 1793
(43) Date of publication of application: 09.03.1994
(73) Proprietor: CENTRO de IMMUNOLOGIA MOLECULAR, Ciudad de la Habana (CU)
(72) Inventor: Fernández Ordonez, Alicia, Calle Rabi No.803 altos, 10 de Octubre, Ciudad de la Habana (CU); Mateo de Acosta del Rio,, Cristina Maria, municipio Boyeros, Ciudad de la Habana (CU); Macias Abraham, Amparo Emilia, Calle 10 No. 208, Ciudad de la Habana (CU); Macineira Pérez, Pedro Pablo, Plaza, Ciudad de la Habana (CU); Tormo Bravo, Blanca Rosa, Vedado, Ciudad de la Habana (CU); Pérez Rodriguez, Rolando, Ciudad de la Habana (CU)
(74) Representative: Smulders, Theodorus A.H.J., Ir.

(56) References cited:
- CANCER RESEARCH vol. 46, no. 11 , November 1986 , PHILADELPHIA PA, USA pages 5592 - 5598 H. MASUI ET AL. 'Mechanism of antitumor activity in mice for anti-epidermal growth factor receptor monoclonal antibodies with different isotypes.'
- CANCER RESEARCH vol. 47, no. 14 , 15 July 1987 , PHILADELPHIA PA, USA pages 3692 - 3696 U. RODECK ET AL. 'Tumor growth modulation by a monoclonal antibody to the epidermal growth factor receptor: Immunologically mediated and effector cell-independent effects.'
- PROCEEDINGS OF THE NATIONAL ACADEMY OF SCIENCES OF THE USA vol. 89, no. 10 , 15 May 1992 , WASHINGTON DC, USA pages 4285 - 4289 P. CARTER ET AL. 'Humanization of an anti-p185HER2 antibody for human cancer therapy.'
- THE JOURNAL OF BIOLOGICAL CHEMISTRY vol. 265, no. 20 , 15 July 1990 , BALTIMORE MD, USA pages 12059 - 12066 F. WEIS ET AL. 'Regulation of epidermal growth factor receptor signal transduction. Role of gangliosides.'
- THE JOURNAL OF BIOLOGICAL CHEMISTRY vol. 263, no. 13 , 5 May 1988 , BALTIMORE MD, USA pages 6296 - 6301 N. HANAI ET AL. 'A novel ganglioside, de-N-acetyl-GM3 (II3NeuNH2LacCer), acting as a strong promoter for epidermal growth factor receptor kinase and as a stimulator for cell growth.'
- IN VITRO CELLULAR & DEVELOPMENTAL BIOLOGY vol. 28A, no. 11 , November 1992 , GAITHERSBURG MD, USA pages 782 - 786 T. KAWAMOTO ET AL. 'Polymorphonuclear leukocytes-mediated lysis of A431 cells induced by IgG1 mouse anti-epidermal growth factor receptor monoclonal antibodies.'

## Description

### FIELD OF THE INVENTION

This invention is related to the field of monoclonal antibodies and provides a method for selecting hybrids which produce monoclonal antibodies that recognize the Epidermal Growth Factor (EGF)-receptor, an antigen present in normal cells and tumoral cells of epithelial origin. These antibodies exhibit superior properties and can be used for diagnosis, treatment and research, particularly of malignant neoplasms, and other diseases.

This invention is also related to therapeutic and diagnostic compositions using these antibodies.

### DESCRIPTION OF THE PRIOR ART

The Epidermal Growth Factor (EGF), a 53 aminoacid polypeptide of 6 kD molecular weight that stimulates epithelial and mesenchymal cell proliferation, has been considered to be one of the growth factors involved in malignant transformations (Cohen S. and Carpenter G., PNAS USA 72, 1317, 1975). This action is mainly performed via its membrane receptor, a 1,186 aminoacid glycoprotein of 170 kD molecular weight. The Epidermal Growth Factor Receptor (EGF-R) has become the object of growing interest in cancer research (Carpenter G. and Cohen S., Receptor Regulation (B series), Vol. 13, 41, Lefkowitz, Ed. Chapman and Hall).

High levels of EGF-Receptor have been detected in malignant tumors of epithelial origin such as breast, bladder, ovarian, vulva, colonic, lung, brain and esophagus cancers. The role played by EGF and its receptor in regulating tumor growth is unknown, but it has been suggested that the EGF-Receptor expression in tumor cells provides a mechanism for autocrine growth stimulation which leads to uncontrolled proliferation (Schlessinger J., Schreiber A.B., Levi A., Liberman T. and Yarden Y., Crit. Rev. Biochem. 14 (2), 93-111, 1983).

The presence of EGF-R in tumor cells has proven to be an indication of poor prognosis in human breast cancer. It has also been considered that this growth factor receptor could widen the concept of hormone dependency in breast cancer (Perez R., Pascual M.R., Macías A. and Lage A., Breast Cancer Research and Treatment 4, 189-193, 1984).

There are reports of some studies with monoclonal antibodies (MAbs) obtained against the EGF receptor, specifically selected for their antigenic recognition which block the EGF binding to the EGF-R. Preliminary studies with some of these antibodies have shown that the EGF receptor presents preferential accumulation in tumors of epidermoid origin. Therefore, potential value is given to the application of these monoclonal antibodies in the immunodetection and immunotherapy of these tumors (Mendelsohn J., Masui H. and MacLeod C., Proc. Am. Assoc. Can. Res. 26, 287-294, 1985; Gullick W.J., Marsden J.J., Whitle N., Ward B., Borrow L. and Waterfield M.D., Cancer Research 46, 285-292, 1985).

The use of biological response modifiers to design treatment schemes for different diseases constitutes a very attractive alternative. Monoclonal antibodies constitute a group of biomolecules being used for such purposes (Deusch K., Mauthe B., Reiter C., Endres N., Classen M. and Riethmuller G., Proceeding of the International Congress of Immunology, Budapest Hungary, 11, 1992).

Immunotherapy with monoclonal antibodies is being studied by many researchers to test its efficacy in the treatment of different neoplastic localizations (Frodin J.E., Philstedt P., Lefvert A.K. and Mellstedt H., Hybridoma, Vol. 7, No. 4, 1988). Many modalities have been proposed, among them: native, conjugated with drugs, bi-specific, anti-idiotype and human monoclonal antibodies.

The high EGF receptor expression in poorly differentiated epidermoid tumors offers the possibility of considering the antibodies as a route for onco-specific treatment whether native or associated with drugs, toxins or radionuclides; (Vollmar A.M., Banker D.E., Mendelsohn J. and Herschman H.R., J. Cell. Physiol. 131, 418-425, 1987). Clinical trials are in progress for the treatment of malignant tumors with these monoclonal antibodies (Magdelenat H., Delarue J.Y., Mady E., Faillot T., Vega F. and Poisson M., Proceedings of VI Conference of MESTMO International Journal of Tumor Markers, Nice, France, Nov. 1991).

The antibodies against EGF receptor which have been used for treatment have been selected primarily for the antigenic recognition, producing tumor growth inhibition through this action. This inhibition occurs through the mechanism of signal transduction in which the EGF receptor is involved (Mendelsohn J., Kawamoto T., Sato G., Sato D., Schlesinger J., Givol D. and Kris R., US Appl. No. 23988, EP 0359282).

On the other hand some gangliosides have been considered both as EGF receptor phosphorylation inhibitors and cell signal transduction inhibitors resulting in cell proliferation inhibition. This indicates that ganglioside could be considered an effective treatment for different neoplastic localization specially EGF receptor bearing tumors (Davis R.J., J. Biol. Chem. 265, 12O59-12O66, 1990).

Besides the possibility of an autocrine growth control in some tumors, involving their own growth factors on proliferation, has been described. This could suggest the use of this mechanism to inhibit tumor growth via monoclonal antibodies against growth factors, and growth factor receptors, and consider it as another possible therapy for malignant neoplasms (Todaro C.J. et al., Proc. Natl. Acad. Sci. USA 77, 5258-5262, 1980). Moreover, the authors of this invention have obtained experimental evidence of the presence of both EGF and EGF receptors in breast tumors (unpublished data).

Up to now, no one has evaluated the possibility of selecting hybridomas that produce monoclonal antibodies with the desired characteristics of: antigenic recognition, complement-mediated or antibody dependent cellular cytotoxicity, tumor growth inhibition and synergetic effect on proliferation inhibition of certain cell lines by the combination with gangliosides or monoclonal antibodies against EGF. The only aspect which has been evaluated is the possibility of tumor growth inhibition through inhibition of the transduction signal produced through the EGF receptor.

In the methods used up to now, hybrids have been selected according to antigenic recognition and, moreover, the antibodies have been obtained through immunizations with the antigen developed from tumor cell lines and have been selected according to anti-tumoral activity through this antigenic recognition.

Researchers involved in this field have not taken into account the possible antitumoral effect that these monoclonal antibodies could elicit through complement mediated or antibody dependent cytotoxicity, nor the combination with other biomolecules such as gangliosides or growth factors or other antibodies against growth factors which could induce a synergetic effect for inhibiting proliferation in cancer cell lines in combination with the monoclonal antibodies against EGF receptor.

Masui et al., Cancer Research 46(11), 1986, 5592-5598, disclose MAbs against the Epidermal Growth Factor Receptor (EGF-R) which can prevent proliferation of some, but not all cultured tumor cells bearing these EGF receptors.

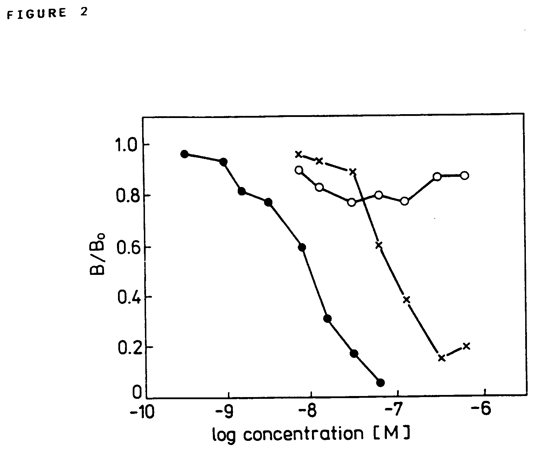

Rodeck et al., Cancer Research 47(14), 1987, 3692-3696, disclose a MAb (MAb425) reacting with EGF receptor on human tumor cells of different tissue origin, but it shows distinctive modulatory effect on the growth of A431 cells. As shown in figure 2 and the discussion on page 3695, MAb425 could stimulate cell growth in some human tumors.

Carter et al., Proc. Nat. Acad. Sci. USA 89(10), May 1992, 4285-4289, disclose the humanized MAb humAb4D5-8 directed against the HER2 oncogene. This tyrosine kinase receptor belongs to the HER family as does the EGF receptor (HER1), but they are different molecules with different physiological relevance in normal embryogenesis and development and also carcinogenesis.

Hanai et al., J. Biol. Chem. 263(13), 1988, 6296-6301, disclose that the ganglioside De-N-acetyl-GM3 acts as a strong promoter for Epidermal Growth Factor Receptor Kinase and as a stimulator for cell growth.

### SUMMARY OF THE INVENTION

This invention provides monoclonal antibodies, and a selection method for hybrids producing them, with qualitatively superior features that may be used to develop diagnostic systems, treatment and research.

According to the previous statement, the main goal of this invention is to provide monoclonal antibodies having superior properties, which recognize the EGF-receptor in human placenta or A-431 cells with high affinity, are capable of inhibiting EGF binding to the receptor, have the ability to produce antibody-dependent cellular cytotoxicity, have the ability to develop complement-mediated cytotoxic activity, which also have the property of inhibiting tumoral growth in certain cell lines and show a synergetic effect to inhibit proliferation of these cell lines when they are used in combination with gangliosides or monoclonal antibodies against EGF.

Another objective of this invention is to provide a selection method for new hybrid cell lines that produce the monoclonal antibodies referred to above.

Another objective is to provide monoclonal antibody-producing hybridomas which meet the selection criteria.

One of the most important objectives of this invention is to provide a pharmaceutical composition containing the monoclonal antibodies against EGF receptor and gangliosides or monoclonal antibodies against EGF, that produces a synergetic effect on inhibiting cell growth. The importance of this preparation could be of remarkable impact in the treatment of lung cancer, any other tumor of epidermoid origin, nervous system origin or any other EGF-Receptor bearing tumor.

Therefore, the invention should provide the aforementioned selection method for obtaining the monoclonal antibodies produced in this way and the compositions these antibodies use, both for diagnosis and treatment.

This invention provides a monoclonal antibody which recognizes human Epidermal Growth Factor (EGF) receptor, is capable of inhibiting EGF binding to the receptor, is capable of inhibiting growth of EGF-dependent tumor cells, and in addition has at least one of the following properties:
a) has antibody-dependent cellular cytotoxicity,
b) has complement-mediated cytotoxic activity,
c) is capable of producing a synergetic effect in inhibiting proliferation of tumor cells if combined with a ganglioside,
d) is capable of producing a synergetic effect in inhibiting proliferation of tumor cells if combined with a monoclonal antibody against EGF,
   or a derivative of said monoclonal antibody selected from humanized, chimeric, reshaped, bispecific, conjugated and anti-idiotypic forms of it,
   or a fragment of said monoclonal antibody or said derivative selected from Fv, Fab, Fab' and F(ab')2 fragments of it.

According to the invention, a monoclonal antibody which has antibody-dependent cellular cytotoxicity and complement-mediated cytotoxic activity is preferred. Preferably, said monoclonal antibody is furthermore capable of producing a synergetic effect in inhibiting proliferation of tumor cells if combined with a ganglioside, and/or capable of producing a synergetic effect in inhibiting proliferation of tumor cells if combined with a monoclonal antibody against EGF.

According to the invention, it is preferred that the monoclonal antibody recognizes both human EGF receptor present in normal cells and EGF receptor present in tumoral cells, most preferably recognizes both human placental EGF receptor and EGF receptor from the A-431 tumor cell line.

According to the invention, it is preferred that the monoclonal antibody is capable of inhibiting the growth of the EGF-dependent lung tumor cell lines U-1752 and/or H-125; that it has an antibody-dependent cellular cytotoxicity in a test using the A-431 cell line as target cells; and that it has a complement-mediated cytotoxic activity in a test using the A-431 cell line as target cells.

Preferred is a monoclonal antibody which is capable of producing a synergetic effect in inhibiting proliferation of tumor cells (such as H-125 cells) if combined with the ganglioside N-acetyl GM3, and which is capable of producing a synergetic effect in inhibiting proliferation of tumor cells (such as H-125 cells) if combined with an anti-EGF monoclonal antibody.

Most preferably, the monoclonal antibody of this invention is an immunoglobuline of the IgG 2a-type.

This invention furthermore provides a composition comprising a monoclonal antibody as defined herein, together with a carrier, a diluent or an excipient therefor. In particular, the invention provides a pharmaceutical composition for use in a therapeutical or prophylactic treatment of malignant neoplasms or pre-neoplastic diseases, comprising a therapeutically or prophylactically effective amount of a monoclonal antibody as defined herein, together with a carrier, a diluent or an excipient therefor. In a most preferred embodiment of the invention, said pharmaceutical composition contains a ganglioside and/or a monoclonal antibody against EGF.

This invention furthermore provides a process for preparing a monoclonal antibody which recognizes human EGF-R, comprising the steps of immunizing a test animal with human EGF-R or an antigenic derivative or fragment thereof, isolating antibody-producing cells from the immunized animal, immortalizing said antibody-producing cells, selecting from said immortalized cells a cell which produces a monoclonal antibody which recognizes human EGF-R, is capable of inhibiting EGF binding to the receptor, is capable of inhibiting growth of EGF-dependent tumor cells, and in addition has at least one of the following properties:
a) has antibody-dependent cellular cytotoxicity,
b) has complement-mediated cytotoxic activity,
c) is capable of producing a synergetic effect in inhibiting proliferation of tumor cells if combined with a ganglioside,
d) is capable of producing a synergetic effect in inhibiting proliferation of tumor cells if combined with a monoclonal antibody against EGF,
   and isolating monoclonal antibody produced by said selected cells.

Preferably, the step of immortalizing the antibody-producing cells isolated from the immunized animal is carried out by fusing the cells with myeloma cells to produce hybridoma cells. The test animal used in the immunisation step preferably is a rodent animal, most preferably a mouse or rat. In the process, the test animal is immunized preferably with human placenta EGF-R.

According to the invention, it is preferred that immortalized cells producing the selected monoclonal antibody are cultivated, either in vitro in a culture medium of the proper tissue, or in vivo in a histocompatible body fluid, or in fermenters such as production medium, followed by separation of the immortalized cells from said medium. The monoclonal antibody produced may then be purified and optionally derivatized or fragmented.

This invention also provides an immortalized cell producing a monoclonal antibody as defined herein, and comprises the use of said monoclonal antibody.

In a typical embodiment, this invention consists of immunizing mice with a partially purified fraction of human placenta epidermic growth factor receptor; removing their spleens and preparing a cell suspension; fusing the spleen cells with myeloma cells in the presence of a fusion promoter; diluting and cultivating the cells; evaluating the supernatant which contains the hybridoma; selecting and cloning a hybridoma which produces antibodies with the following characteristics: recognition of the EGF receptor, with high affinity, and with the ability to inhibit EGF binding to the receptor, the capability of producing antibody-dependent cellular cytotoxicity (ADCC), complement-mediated cytotoxic activity, inhibition of tumoral growth in certain cell lines and show a synergetic effect in proliferation inhibition of these cell lines in combination with gangliosides or monoclonal antibodies against EGF; recover the antibody from the supernatant liquid and collect the malignant ascites or the serum which contains the desired antibodies.

The selection, preparation and characterization of the hybrid cells (hybridomas) and the resulting antibodies will be better understood with the following description and examples.

### DETAILED DESCRIPTION OF THE INVENTION

The hybridoma preparation method generally includes the stages described below, which includes the methods used for the selection of hybridomas.
A. Mice immunization with a partially purified fraction of human placental EGF-R. Although it has been verified that female Balb/c mice are best, other breeds of mice may be used. The immunization program and antigen concentration must be carefully measured to produce useful amounts of adequately stimulated splenocytes. One method found to work well was: 4 immunizations at 0, 14, 21 and 28 day intervals with 25 µg of the immunogen per mouse in 0.2 mL of phosphate buffered saline solution, with complete adjuvant on day 0 and incomplete adjuvant on days 14, 21 and 28. The last inoculation was 50 µg applied intravenously, previous to cellular fusion.
B. Removal of the spleen of each immunized mouse and preparation of a spleen cell suspension using the adequate medium. Approximately 50 mL of medium per spleen is adequate according to known experimental techniques.
C. Fusion of the spleen cells suspension with murine myeloma cells from an adequate line under the effect of a fusion agent. The preferred ratio is 10 spleen cells per myeloma cell. An approximate total volume of 0.5-1 mL of fusion medium to every 10⁸ cells is appropriate. Many murine myeloma cell lines are recognized and available from the academic community or specialized banks. The cell line used, however, should be of the so-called "drug-resistant" type so that the fused myeloma cells will not survive in a selected medium, but the hybrids do. The most commonly used classes are the cell lines resistant to 8-azaguanine, that lack the hypoxanthine-guanine-phosphoribosyl-transferase enzyme and, therefore, will not survive in a HAT medium (hypoxanthine-aminopterin and thymidine). It is also generally preferable to use a myeloma line of the class known as "non-secreting", as these do not produce antibodies on their own. If necessary, however, classes that secrete may also be used.
   Although the preferred fusion promoting agent is polyethylene glycol with an average molecular weight of approximately 1000 to 4000, other promoters may also be used.
D. Dilute and cultivate in the plate wells, the mixture of non-fused spleen cells, non-fused myeloma cells and fused cells in a selective medium that does not maintain the non-fused myeloma cells (i.e. HAT). After a week, the non-fused spleen cells cease to reproduce. The fused cells, on the other hand, continue to reproduce as a result of the malignant characteristics inherited from the parental myeloma and the survival capacity of parental spleen cells in the selective environment.
E. METHODS OF HYBRIDOMA SELECTION
   1. Evaluate the hybridoma supernatant in each well to determine the presence of the antibodies which selectively recognize with high affinity the human placenta EGF receptor, and the receptor present in the A-431 tumoral cell line and these monoclonal antibodies are also capable of inhibiting EGF binding to the receptor. This evaluation can be made according to the preferred techniques of either radio-receptor or immunoassay using as antigens the cell lines rich in EGF receptor and the receptor from human placenta which has been partially purified according to affinity purification methods described below.
   2. Evaluate the hybridoma supernatant in each well, to determine the presence of the antibodies which have the properties of inhibiting tumoral growth of lung cancer cell lines. Different concentrations of the monoclonal antibody are added to the cells in suspension and a comparison is made with a control group one without an antibody and other with an irrelevant antibody. Measurements of protein concentration are performed with Lowry Rosebrough method, but any other method for measuring total protein concentration could be used.
   3. Evaluate the hybridoma supernatant in each well to determine the presence of the antibodies which have the properties to produce antibody-dependent cellular cytotoxicity, using the standard measurement method for this activity, which is carried out through target cells labeled with ⁵¹Cr, effector cells and different concentrations of the monoclonal antibody.
   4. Evaluate the hybridoma supernatant in each well to determine the presence of the antibodies which have the properties to produce complement-mediated cytotoxic activity. This is done with standard methods for measuring this activity, using target cells labeled with ⁵¹Cr, rabbit complement and different concentrations of the monoclonal antibody.
   5. Evaluate the hybridoma supernatant in each well to determine the presence of the antibodies which have the property to produce synergetic effect in cell proliferation inhibition when they are combined with gangliosides.
      A cellular suspension is obtained and resuspended in culture medium and the gangliosides added at a concentration range of 0.5-50 microMoles. Then the monoclonal antibodies (supernatant) are added in a concentration range of 10 to 100 micrograms per mL. At the third and sixth day total protein concentration is measured, Lowry Rosebrough method is preferably used, but any other method could be used.
   6. Evaluate the hybridoma supernatant in each well to determine the presence of the antibodies which have the property to produce synergetic effect in cell proliferation inhibition when they are combined with a monoclonal antibody against EGF.

   A cellular suspension can be obtained and resuspended in culture medium and the MAb against EGF is added at an adequate concentration range and the monoclonal antibodies are added in a concentration range of 10 to 100 micrograms per mL. At the third and sixth day total protein concentration is measured, Lowry Rosebrough method is preferably used, but any other method for measuring total protein concentration could be used.
F. Select (i.e. via limiting dilution) and clone the hybridoma that produces the antibodies.
   Once the desired hybridoma has been selected and cloned at least twice, the antibodies may be produced using either of two methods:
   a) The monoclonal antibodies may be produced through in vitro cultivation of the hybridoma in an adequate medium for the indicated time, followed by the recovery of the desired antibody from the supernatant. The proper medium and time are known or easily determined. This in vitro technique produces an essentially monospecific antibody that is fundamentally exempt of other anti-human specific immunoglobulins. There is a small quantity of other immunoglobulins as the medium contains xenogenic serum (fetal calf or newborn calf serum). This in vitro method can be scaled up in bio-reactors to obtain the appropriate amounts of the desired monoclonal antibodies.
   b) The other production method involves injecting the hybridoma in preferably syngenic mice. Following an appropriate incubation period the hybridoma will cause the formation of non-solid tumors that produce antibodies. These will provide a high concentration of the desired antibodies (5 - 20 mg/mL) in the blood stream and the peritoneal exudate (ascites) of the murine host. Although these hosts also present normal antibodies in blood and ascites, the concentration of monoclonal antibodies is higher. Furthermore, as the normal antibodies are not anti-human specific, the monoclonal antibodies obtained are essentially free of any anti-human immunoglobulin contaminant. The immunoglobulins produced by the incorporation of light myeloma chains are non-specific irrelevant peptides that merely dilute the monoclonal antibodies without affecting their specificity.

   In agreement with this aspect, the invention provides hybridoma cell lines capable of producing monoclonal antibodies which recognize the EGF receptor with high affinity, capable of inhibiting EGF binding to the receptor, capable of producing antibody-dependent cellular cytotoxicity, capable of developing complement-mediated cytotoxic activity, that can inhibit tumoral growth in some cell lines and in combination with gangliosides or monoclonal antibodies against EGF produce a synergetic effect in proliferation inhibition of them.
   Said antibodies could be used to develop immuno-detection techniques, develop new treatments with bio-molecules, study the antigen's molecular topography, develop anti-idiotypical and humanized antibodies, study their use in idiotypical regulation of the immune network and in immuno-purification and structural characterization of the antigen they recognize.
G. OBTENTION OF A THERAPEUTIC COMPOSITION WITH THE MONOCLONAL ANTIBODIES WHICH HAVE BEEN OBTAINED WHICH ELICIT IMMUNOLOGICAL EFFECT
   Once the purified antibodies have been obtained, a therapeutic composition is developed which contains an effective quantity of any monoclonal antibodies produced by the hybridoma obtained through the Hybridoma Selection Method, and which elicit immunological effect. The antibodies selected for this condition were characterized by the complement mediated cytotoxic activity and antibody dependent cellular cytotoxicity. This was demonstrated using the standard techniques; A-431 cell line labeled with ⁵¹Cr was used as target cells. The target cells in an adequate concentration were pipetted on microplates with the monoclonal antibody. The complement source could be human or rabbit serum in culture medium. The incubation of H-125 cell lines with the MAb showed in the presence of complement, a cytotoxic effect. The cell cytotoxicity mediated by antibodies was carried out using Balb/c mouse peritoneal macrophages as effector cells, allowed the obtention of a cytolytic effect.
   In this preparation a pharmaceutical vehicle should be used, which may be any of those used in conventional pharmacopeia, or maltose, or trehalose, saccharose or polyethylene glycol 800 or the combination of these with the Zn cation, in different concentrations. This preparation could be from said antibody or from its fragments (Fv, Fab, Fab', F(ab')2), or from the humanized, chimerical, "reshaped", bispecific antibody or any of the aforementioned antibodies conjugated with toxins, radionuclides, or any other available drug, or it may be from the anti-idiotypic antibodies (AB2, AB·) obtained against said antibody; for the treatment or prevention of malignant neoplasms or pre-neoplastic diseases, especially in central nervous system tumors, tumors of the head and neck, and breast and lung adenocarcinomas.
H. OBTENTION OF A THERAPEUTIC COMPOSITION FROM THE MONOCLONAL ANTIBODIES WHICH HAVE BEEN OBTAINED IN COMBINATION WITH A GANGLIOSIDE
   Once the purified antibodies have been obtained, a therapeutic composition is developed which contains an effective quantity of any monoclonal antibodies produced by the hybridoma obtained through the Hybridoma Selection Method, including an adequate concentration of a ganglioside.
   The synergetic effect demonstrated by the combination of these monoclonal antibodies and different gangliosides has been shown. Different concentrations of gangliosides could produced growth inhibition of cell proliferation when they are combined with monoclonal antibodies against EGF receptor.
   A cellular suspension is obtained from H-125 and U-1752 cell lines, respectively and is resuspended in culture medium. Gangliosides, in a concentration range of 0.5 - 50 microMoles are added and the monoclonal antibodies against EGF-Receptor are added in a concentration range of 10 to 100 micrograms per mL.
   At the third and sixth day total protein concentration is measured by Lowry Rosebrough method (Lowry D.H., J. Biol. Chem. 193: 265, 1951), but any other method could be used. A synergetic effect of the combination Ganglioside - monoclonal antibody against EGF receptor is observed in inhibiting proliferation of a lung cancer cell line in relation to the controls used in this test.
   There was almost 100% inhibition of proliferation in relation to control; the effect produced by the combination is greater than the simple additive effect of both molecules independently.
   In this preparation a pharmaceutical vehicle should be used, which may be any of those used in conventional pharmacopeia, or maltose, or trehalose, saccharose or polyethylene glycol 800 or the combination of these with the Zn cation, in different concentrations. This preparation could be from said antibody or from its fragments (Fv, Fab, Fab', F(ab')2),or from the humanized, chimerical, "reshaped", bispecific antibody or any of the aforementioned antibody conjugated with toxins, radionuclides, or any other available drug, or it may be from the anti-idiotypic antibodies (AB2, AB·) obtained against said antibodies; for the treatment or prevention of malignant neoplasms or pre-neoplastic diseases, especially in central nervous system tumors, tumors of the head and neck, and breast and lung adenocarcinomas.
I. OBTENTION OF A THERAPEUTIC COMPOSITION FROM THE MONOCLONAL ANTIBODIES WHICH HAVE BEEN OBTAINED IN COMBINATION WITH MONOCLONAL ANTIBODIES AGAINST EGF
   Once the purified antibodies have been obtained, a therapeutic composition is developed which contains an effective quantity of any monoclonal antibodies produced by the hybridoma obtained by the Hybridoma Selection Method, including an adequate concentration of a monoclonal antibody against EGF, and a pharmaceutical vehicle, which may be any of those used in conventional pharmacopeia, or maltose, or trehalose, saccharose or polyethylene glycol 800 or the combination of these with the Zn cation, in different concentrations. This preparation could be from said antibody or from its fragments (Fv, Fab, Fab', F(ab')2), or from the humanized, chimerical, "reshaped", bispecific antibody or any of the aforementioned antibodies conjugated with toxins, radionuclides, or any other available drug, or it may be from the anti-idiotypic antibodies (AB2, AB·) obtained against said antibodies; for the treatment or prevention of malignant neoplasms or pre-neoplastic diseases, especially in central nervous system tumors, tumors of the head and neck, and breast and lung adenocarcinomas.

### EXAMPLE 1

### SELECTION METHODS FOR HYBRIDOMAS

### a) Selection of the Hybridoma According to the Antigenic Recognition Method

The hybridoma supernatant in each well was evaluated, to determine the presence of antibodies which selectively recognize with high affinity the human placenta EGF receptor and the receptor from the A-431 cell line of epidermoid origin and are also capable of inhibiting EGF union with the receptor.

After the cellular fusion, the cells were cultured in selective HAT medium (hypoxanthine, aminopterin and thymidine) at 37°C, with a 5% CO₂ moist atmosphere.

Three weeks later, 10 µL of supernatant from the cultures which contain hybridomas were added to the wells of polyvinyl chloride plates which had been previously coated with extracts of placental membrane or from the A-431 cell line, which contain EGF receptor. Detection of the antibodies which react with the antigen expressed in these membrane extracts was made through a solid phase, indirect, immuno-enzymatic assay. The antigen was immobilized by coating the polyvinyl chloride plates with partially purified, soluble EGF receptor. HEPES 10 mM, pH 7.5 buffer solution was used. The probe used as a second antibody was peroxidase conjugated anti-mouse antibody (Amersham).

Hybridoma cultures which contain antibodies that react to membrane extracts containing the receptor, are selected and they are cloned twice according to the limiting dilution method, in the presence of conditioning cells.

The method has the special characteristic of simultaneously select the antibodies for both antigens: the receptor from human placenta, which possesses normal epitopes, and the receptor from the A-431 cell line, which epitopes appears during the malignant progression, which means that the epitope selected in the antibody by the Selection Method is preserved in the receptor molecules present in normal tissues and also appears in most of the receptor molecules present in tumoral tissue of epidermoid origin.

### b) Selection of the Hybridoma According to Anti-Tumoral Activity

The hybridoma supernatant in each well was evaluated to determine the presence of the antibodies which inhibit tumoral growth in lung cancer cell lines U-1752 and H-125 (NCI, Bethesda, Maryland). These cell lines show an EGF dependence for their proliferation.

The culture medium used was RPMI-1640 (Gibco, USA) supplemented with 5-10% calf serum. The cells were kept in incubators at 37°C in a humid atmosphere and carbon-dioxide air (95%-5%).

The monoclonal antibody's inhibitory effect was measured in the cells in trypsin suspension and re-suspended at concentrations of 2.5 x 10⁴ cells/mL of culture medium to which different concentrations of monoclonal antibody were added: 10, 30 and 100 µg/mL. A control group with an irrelevant antibody was also studied in concentrations of 100 µg/mL. The cellular suspensions were placed in 24-well culture plates (1 mL per well), and each sample was tested three times. Plates were prepared for each cellular line and analyzed at different exposure times (1, 3, 5 and 7 days). The determination of the total number of cells was made according to the quantitative method of total protein determination (D.H. Lowry).

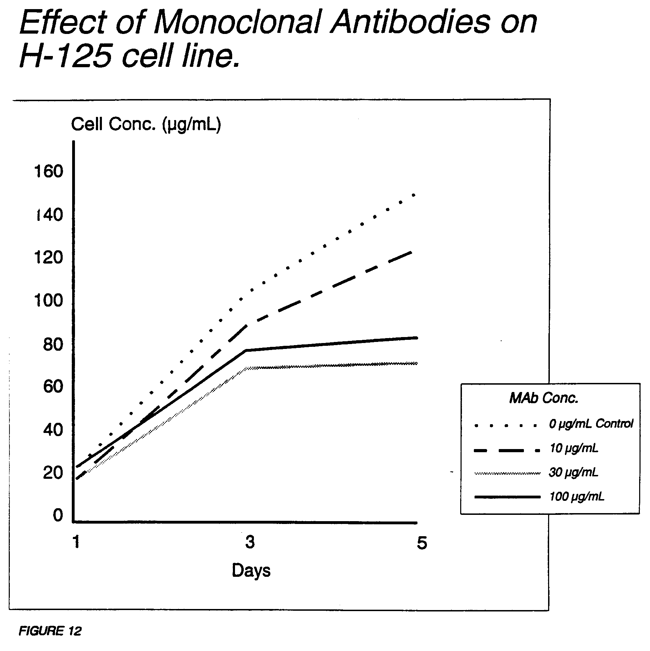

Inhibition of the H-125 and U-1752 lung cancer cell line proliferation was produced in 50% in relation to the control, this result can be observed in Figure 12.

This part of the selection method has the particular characteristic that the hybridomas selected produce monoclonal antibodies with a specific recognition of the antigen and simultaneously present the ability to inhibit tumoral growth.

### c) Selection of the Hybridoma According to the Capacity to Produce Antibody-Dependent Cellular Cytotoxicity

The hybridoma supernatant in each well was evaluated to determine the presence of the antibodies which produce antibody-dependent cellular cytotoxicity. A standard measurement technique for this activity was used; A-431 cell line labeled with ⁵¹Cr (100 µCi) was used as targets.

The effector cells were blood cells from normal adults prepared with a Ficoll-Hypaque gradient in which the mononuclear cells from the peripheric blood were separated, washed three times and adjusted in an EFFECTOR CELL/TARGET CELL ratio of 200:1.

The target cells in 10⁴ concentration were pipetted into 96-well plates which contain 25 µL of monoclonal antibody (ascitic liquid diluted 1/10). The plates with the target cells and the antibody were incubated at 4°C for 30 minutes. The effector cells (100 µL) were added to the plate and centrifuged immediately 100 x g for 2 minutes. They were then incubated during four hours in a humid chamber at 37°C with 5-95% CO₂ air. From this experiment it could be demonstrated an antibody dependent inhibitory effect of 22%.

In this part of the selection method, monoclonal antibodies producing hybridomas have already been obtained which simultaneously possess three properties: antigenic recognition, inhibition of tumoral growth and at the same time, antibody-dependent cellular cytotoxicity.

### d) Selection of the Hybridoma According to the ability to Produce Complement-Mediated Cytotoxic Activity

The supernatant in each well was evaluated to determined the presence of the antibodies which produces complement-mediated cytotoxic activity, using the standard measurement method and an A-431 cell line labeled with ⁵¹Cr (100 µCi) was used as target cells.

The target cells in a concentration of 10⁴ were pipetted into 96-well plates which contain 25 µL of monoclonal antibody (ascitic liquid diluted 1/10).

The complement source consisted of 100 µL of rabbit serum at 25% in culture medium. It was incubated for four hours in a humid chamber at 37°C with 5-95% CO₂ air. The plates were later centrifuged and the counts per minute (cpm) were measured in 100 µL of the supernatant in a gamma counter.

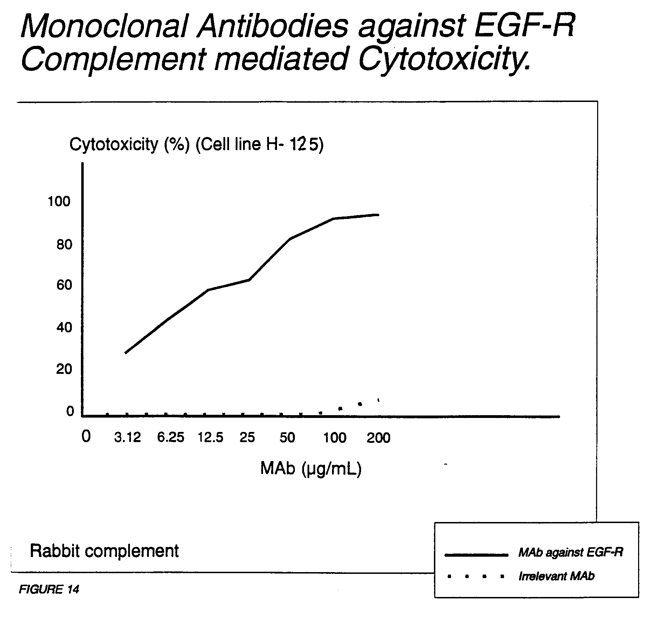

Several working solutions of the antibodies were tested to verify what was the maximum toxicity value. In this experiment a 60% complement mediated inhibitory effect was demonstrated as it is shown in Figure 14.

### e) Selection of the Hybridoma According to the ability to Produce a synergetic effect in combination with N-acetyl GM3

The H-125 cell line expressing 10⁴ - 10⁵ EGF receptor binding sites and classified as lung adenocarcinoma was used as the experimental model for the screening. A cellular suspension was obtained with 10⁴ - 10⁵ cells per mL and was resuspended in culture medium. N-acetyl GM3 is added then at a concentration of 5 micrograms and a monoclonal antibody against EGF Receptor is added at a concentration of 30 micrograms per mL. At the third and sixth day total protein concentration was measured by Lowry Rosebrough method.

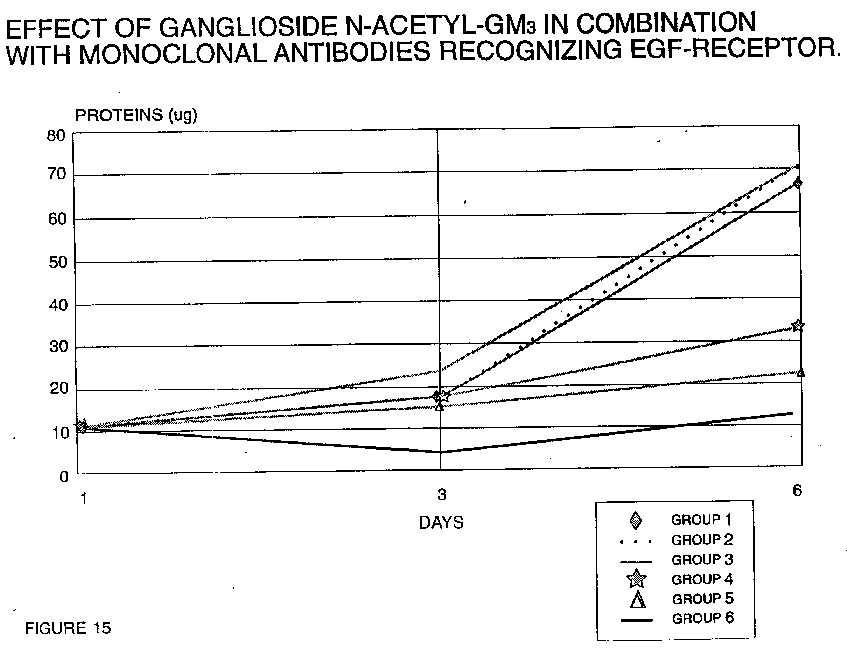

A synergetic effect of the combination N-acetyl GM3-monoclonal antibody against EGF receptor was observed in inhibiting proliferation of a lung cancer cell line in relation to the controls used in this test. There was almost 100% inhibition of proliferation in relation to control. So the effect produced by the combination is much more than the simple additive effect of both molecules independently. This result is observed in Figure 15.

### f) Selection of the Hybridoma According to the ability to Produce a synergetic effect in combination with monoclonal antibodies against EGF

The H-125 cell line expressing 10⁴ -10⁵ EGF receptor binding sites and classified as lung adenocarcinoma was used as the experimental model for the screening. A cellular suspension was obtained with 10⁴ - 10⁵ cells per mL and was resuspended in culture medium. The MAbs against EGF are added at a concentration of 5 microMoles and monoclonal antibodies against EGF-Receptor are added at a concentration of 30 micrograms per mL. At the third and sixth day total protein concentration was measured by Lowry Rosebrough method.

A synergetic effect of the combination MAbs against EGF - monoclonal antibody against EGF receptor was observed in inhibiting proliferation of a lung cancer cell line in relation to the controls used in this test.

The antibodies produced by the hybridomas based on the selection method used have superior properties which give them the distinction of: high affinity antigenic recognition for the EGF Receptor; antibody-dependent cellular cytotoxicity; complement-mediated cytotoxic activity; the capacity of inhibiting the tumoral growth in cell lines and a synergetic effect in combination with N acetyl GM3 or MAbs against EGF.

### EXAMPLE 2

### BIOCHEMICAL CHARACTERIZATION OF THE MONOCLONAL ANTIBODIES OBTAINED BY THE SELECTION PROCEDURE

Monoclonal antibodies obtained are IgG2a -type immunoglobulins secreted by the hybridomas obtained from the fusion of SP2/Ag14 mouse myeloma with Balb/c mouse spleen lymphocytes immunized with a partially purified fraction of Human placenta EGF receptor.

The purification of solubilized EGF receptor was performed by affinity chromatography. Briefly: the placenta crude membrane fractions were solubilized in Tris-HCl 20 mM buffer, pH 7.4, glycerin 10% and Triton X-100 1% solution during 1 hour at room temperature. The homogenate was centrifuged at 10,000 g. The supernatant was applied to an EGF affinity matrix. The EGF enriched fraction was eluted with ethanolamine 5 mM buffer, pH 9.7 with glycerin 10% and Triton X-100 0.1% solution. This solubilized EGF receptor enriched fraction has the capacity to bind ¹²⁵I-EGF, and showed a major 170 kD band in SDS-PAGE. One hundred grams were processed each time. Average yield was 15-25 µg of partially purified EGF receptor per gram of wet tissue.

These monoclonal antibodies possess the following characteristics:

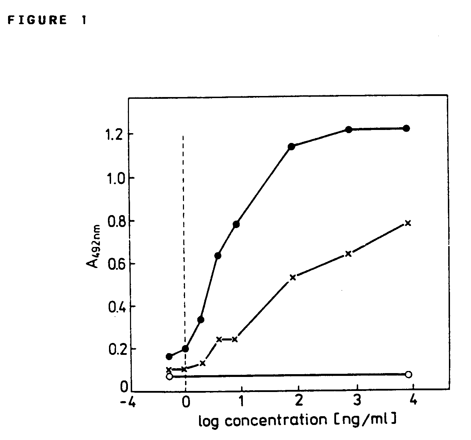

A high binding affinity for the receptor with Kd values of 10⁻⁹ M calculated through the semi-saturated concentration curve obtained, as it is shown in Figure 1, this affinity is similar to the receptor affinity to EGF.

They are capable of inhibiting the ¹²⁵I-EGF's binding to the membrane receptor using a microsomal fraction of human placenta, (Figure 2 and Figure 4) it is possible to deduce from its 10⁻⁸ M Ki values, that the binding site is not the same as that of Epidermal Growth Factor, and that the inhibition is provoked by a conformational or steric effect. These monoclonal antibodies suppress the EGF's effect on the A431 cell line, even in the presence of synergetic agents such as gamma-IFN (Figure 6 and 7). These MAbs also inhibit EGF-dependent clonogenic growth of NRK cells and inhibit EGF-receptor dependent auto-phosphorylation (Figure 8) considered to be a crucial step in the transduction of the cellular proliferation signals, which demonstrates the EGF-antagonistic role played by these monoclonal antibodies.

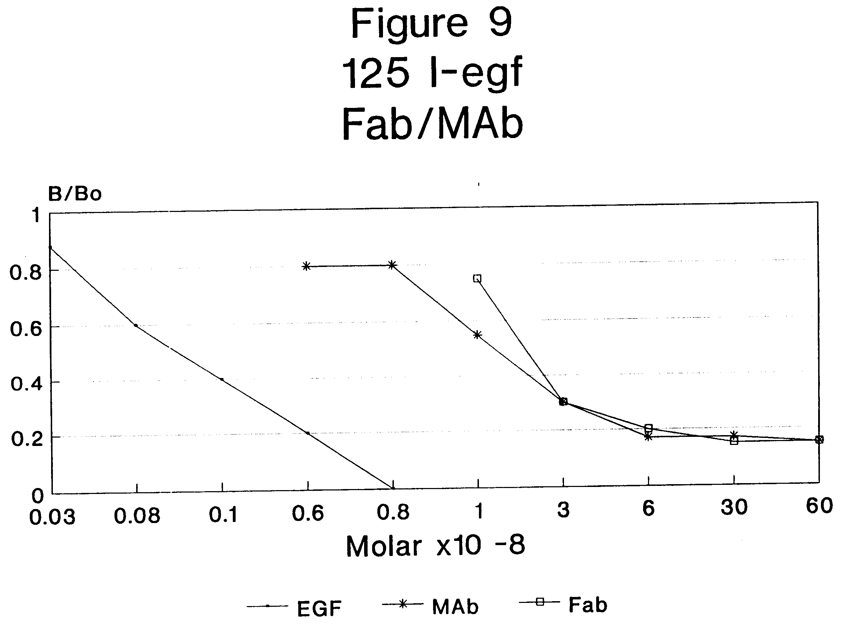

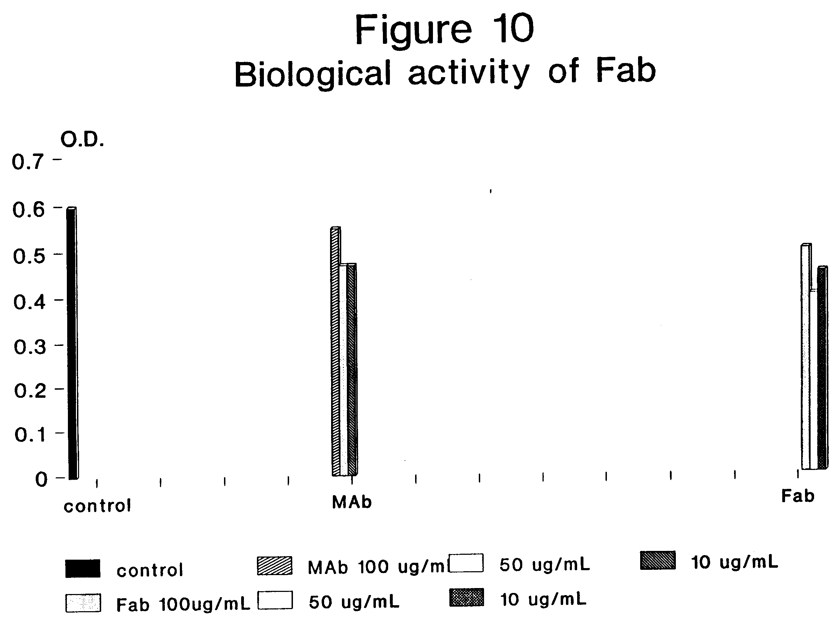

Studies that corroborate this effect were carried out with the Mab and its Fab fragment obtained by papain digestion (Figure 9 and 10). This effect proved to be dose-dependent. The fragment retains the same ability to suppress EGF cellular proliferation.

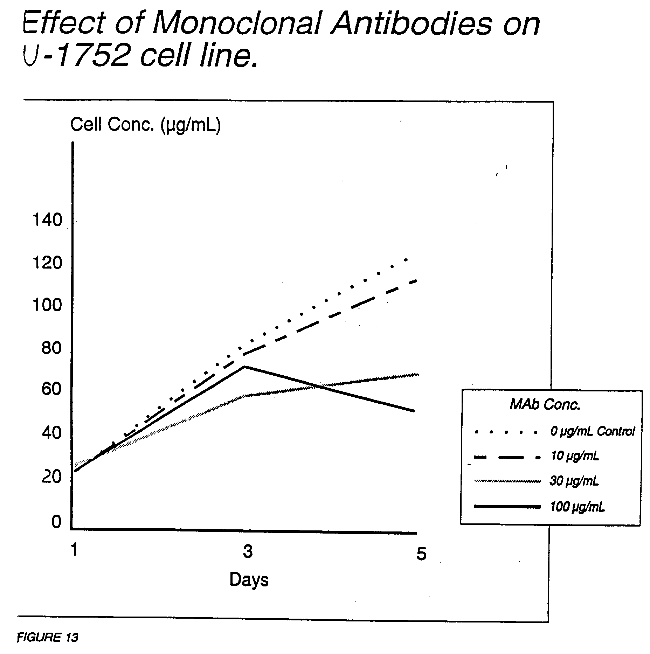

The capacity of these monoclonal antibodies for inhibition of the proliferation of different cellular lines has also been shown. In the lung cancer cell lines studied (H-125 and U-1752) it was found that in concentrations of 10 µg/mL of these antibodies, growth was slightly decreased and that there was no significant difference in comparison to the control, however, when it was applied in concentrations of 30 µg/mL, growth inhibition was achieved in the culture by day 3 which remained stationary and on the fifth day, an inhibition of 50% compared to the control was observed (Figures 12 and 13).

These monoclonal antibodies are also characterized by complement-mediated cytotoxic activity and antibody-dependent cellular cytotoxicity.

The incubation of the H-125 cellular line with the monoclonal antibodies showed, in the presence of rabbit complement, a cytotoxic effect of 60% at a concentration of 12.5 µg/mL (Figure 14). The cellular cytotoxicity mediated by antibodies carried out using Balb/c mouse peritoneal macrophages as effector cells with 110:1 ratio (Effector : Target) produced a cytolytic effect of 22%.

The synergetic effect demonstrated by the combination of these monoclonal antibodies and different concentrations of gangliosides, was also shown. This combination can produce a growth inhibition of cell proliferation. N-acetyl GM3 was added in a concentration of 5 micromoles and the MAb was added in concentration of 30 micrograms per mL.

A synergetic effect of the combination N-acetyl GM3-monoclonal antibody against EGF receptor is observed in inhibiting proliferation of a lung cancer cell line in relation to the controls used in this test.

There was almost 100% inhibition of proliferation in relation to control; the effect produced by the combination is greater than the simple additive effect of both molecules independently (Figure 15).

A study of the sequence of the variable regions of the heavy chain of the immunoglobulins obtained through the Polymerase Chain Reaction (PCR) was performed. Amplifications of at least 2 independent PCR samples and at least two independent pUC19 clones were used to determine the sequence. Once the variable regions of the light and heavy chain were amplified, they were cloned in the pUC19 vector sequence and the heavy chain sequence was identified.

According to the Kabat Database, this sequence has been classified of the miscellaneous group.

The re-ordering of the variable regions of the heavy chain defines the epitope of the EGF receptor which is recognized by these monoclonal antibodies.

### EXAMPLE 3

### CHARACTERIZATION OF THE REACTIVITY OF THE MONOCLONAL ANTIBODIES OBTAINED BY THE SELECTION PROCEDURE

Fresh tissues were selected for the study, these included samples of heart, prostate, liver, lung, kidney, spleen, ovary, pancreas, brain, testis, adrenal glands, hypophysis, lymph nodes, medulla, thyroid, tonsils, stomach, small intestine, colon, skin, submaxillary gland, placenta and cerebellum. Several nerve tissues, hematopoietic and sarcomatous tumors were also studied.

The fresh tissues were frozen in liquid nitrogen and stored at -20°C. Histopathological study was evaluated using hematoxylineosin stained sections. Consecutive sections based on the histopathologically evaluated blocks were used for staining with the immunoperoxidase technique.

The tissue reactivity was demonstrated using the biotin-streptoavidin-peroxidase complex (Amersham, U.K.).

The study includes sections with positive (skin sections) and negative (tris buffered saline solution) controls. The reaction with the enzyme produces a brown precipitate and is classified as follows:
* no reaction (-);
* mild reaction (+);
* moderate reaction (++);
* intense reaction (+++).

Fresh normal tissue was taken from recently performed autopsies and tissue reactivity was shown to be intense according to the established pattern in pancreas and testis.

Fresh normal tissue was taken from recently performed autopsies and tissue reactivity was shown to be moderately intense, according to the established pattern, in: placenta, skin, prostate, liver, kidney, ovary, thyroid, intestine and non-lymphoid tonsil tissue (Table 1).

Epidermoid tumors, and central nervous system tumors such as glioma, meningioma, malignant histiocytoma, neuro-fibrosarcoma, as well as mammary and nasopharyngeal tumors have shown intense reaction, according to the established pattern, and in much higher concentration than in normal tissues of the same origin (Table 2).

Detection only occurs in tissue containing the receptor, which demonstrates that the recognition is specific to the molecule's epitope, and therefore its private nature, as well as the absence of related epitopes in other antigens.

### EXAMPLE 4

### DEVELOPMENT OF A THERAPEUTIC COMPOUND WHICH ELICIT IMMUNOLOGICAL EFFECT

A therapeutic compound is developed containing an effective quantity of monoclonal antibody produced by the hybridoma obtained through the Hybridoma Selection Method that produces antibody-dependent cellular cytotoxicity. To do this, we used the standard measurement technique for this activity. A-431 cell line labeled with ⁵¹Cr (100 µCi) was used as targets. The effector cells were blood cells from normal adults prepared with a Ficoll-Hypaque gradient in which the mononuclear cells from the peripheric blood were separated, washed three times and adjusted in a EFFECTOR CELL/TARGET CELL ratio of 200:1.

The plates with the target cells and the antibody were incubated at 4°C for 30 minutes. The effector cells (100 µL) were added to the plate and centrifuged immediately 100 x g for 2 minutes. They were then incubated for four hours in a humid chamber at 37°C with 5-95% CO₂ air. The counts per minute (cpm) were measured in 100 µL of supernatant in a gamma counter.

The complement-mediated cytotoxic activity was measured, using the standard measurement method and using A-431 cell line labeled with ⁵¹Cr radioactive (100 µCi) as target cells.

The target cells in a concentration of 10⁴ were pipetted on 96 - well plates which contain 25 µL of monoclonal antibody (ascitic liquid) diluted 1/10.

The complement source consisted of 100 µL of rabbit serum at 25% in culture medium. It was incubated for four hours in a humid chamber at 37°C with 5-95% CO₂ air. The plates were later centrifuged and the counts per minute were measured in 100 µL of the supernatant in a gamma counter. Several working solutions of the antibody were tested to verify what was the maximum toxicity.

These experiments showed that in the presence of rabbit complement a cytotoxic activity of 59% at a concentration of 12.5 µg/mL, and the cellular cytotoxic effect mediated by antibodies carried out with peritoneal macrophages produced a cytolytic effect of 22%.

This preparation includes a pharmaceutical vehicle which contains 2.55 mg of monobasic sodium phosphate, 9.00 mg of dibasic sodium phosphate, 43.00 mg of sodium chloride, 1 mg of polysorbate 80, 5.00 mL of injection water.

### EXAMPLE 5

### DEVELOPMENT OF A THERAPEUTIC COMPOUND WITH N-ACETYL GM3

A therapeutic compound is developed containing an effective quantity of monoclonal antibody produced by the hybridoma obtained through the Hybridoma Selection method, together with N-acetyl GM3.

A synergetic effect of the combination N-Acetyl GM3 with the monoclonal antibodies was observed in inhibiting proliferation of a lung cancer cell line.

To perform the experiment a cellular suspension was obtained and resuspended in culture medium. N-acetyl GM3 was added at a concentration of 5 microMoles and the MAb was added at a concentration of 30 µg/mL. At the third and sixth days total protein was measured by Lowry method.

There was almost 100% inhibition of proliferation in relation to control but the effect produced was greater than the simple additive effect.

This preparation includes a pharmaceutical vehicle which contains 2.55 mg of monobasic sodium phosphate, 9.00 mg of dibasic sodium phosphate, 43.00 mg of sodium chloride, 1 mg of polysorbate 80, and 5.00 mL of injection water.

### EXAMPLE 6

### DEVELOPMENT OF A THERAPEUTIC COMPOUND WITH MAB AGAINST EGF

A therapeutic compound is developed containing an effective quantity of monoclonal antibody produced by the hybridoma obtained through the Hybridoma Selection Method, together with anti EGF MAb, plus a pharmaceutical vehicle which contains 2.55 mg of monobasic sodium phosphate, 9.00 mg of dibasic sodium phosphate, 43.00 mg of sodium chloride, 1 mg of polysorbate 80, 5.00 mL of injection water.

### BRIEF DESCRIPTION OF THE DRAWINGS

- Figure 1-: shows the saturation curves obtained during solid-phase ELISA for placental membrane and A-431 cell fractions. The axis of the abscissa reflects the log. of the antibody concentration. An irrelevant antibody was used as negative control (antibody concentrations were from 0.5 to 7500 ng/mL).
- Figure 2-: shows the displacement curves obtained from Radio Receptor Analysis. The competence assay was performed using microsomal fractions of human placenta and different concentrations of the antibody and a negative control (anti-cytokeratin monoclonal antibody). The EGF displacement curve was used as positive control.

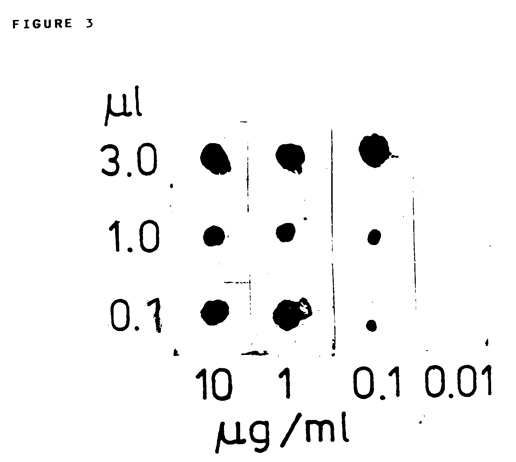

- Figure 3-: shows the monoclonal antibody immuno-reactivity assay (dot blot) with the A-431 cell purified EGF-R. Aliquots (3.0, 1.0, 0.1 µL) of the purified EGF-R (30 ng/mL) were applied in the nitrocellulose and incubated with different concentrations of the antibody.

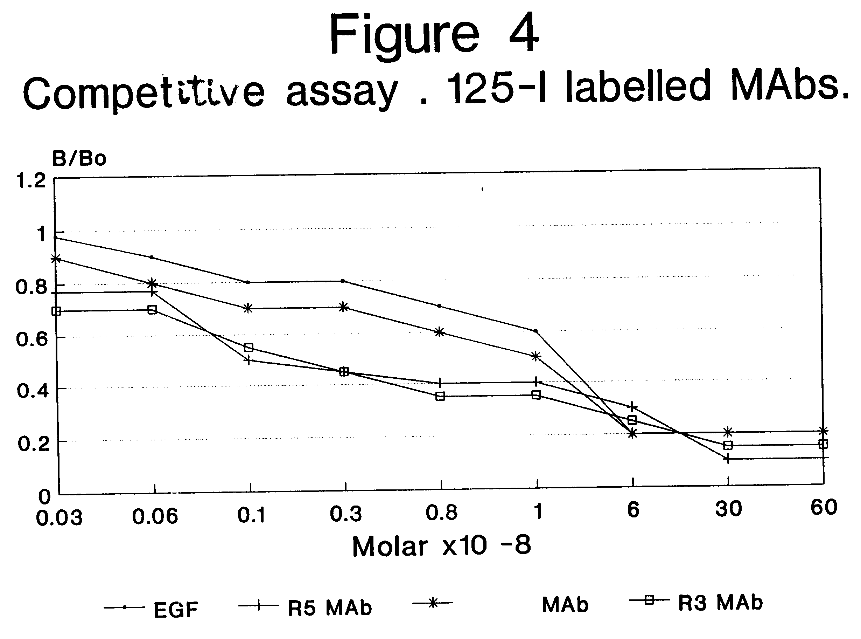

- Figure 4-: shows a competitive assay among different monoclonal antibodies against the EGF Receptor, labeled with 125-Iodine.

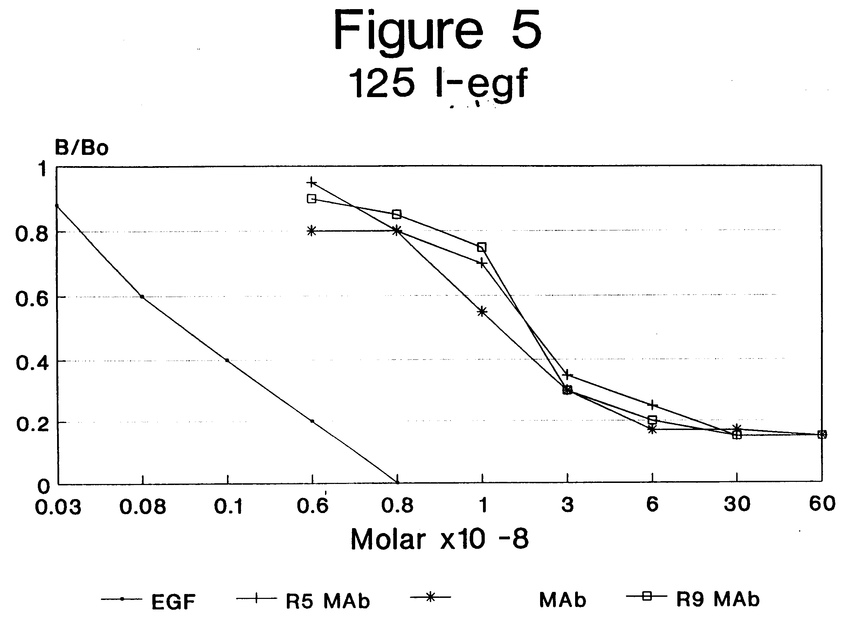

- Figure 5-: shows a competitive assay with a microsomal fraction of human placenta and different concentrations of the monoclonal antibodies obtained against the EGF-R. EGF labeled with 125-Iodine was used as the radio tracer.

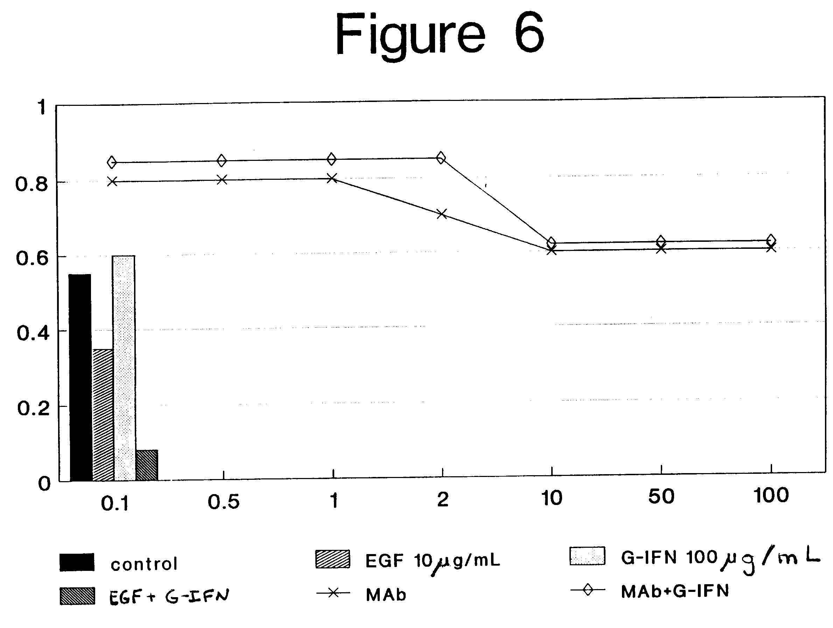

- Figure 6-: shows the monoclonal antibody's effect on A-431 cells. The cells were cultured with EGF (1 µg/mL); Gamma-IFN (100 µg/mL); EGF + G-IFN; and different concentrations of the monoclonal antibody alone or combined with Gamma-IFN. Determination of the proteins in the cellular monolayer was upon the seventh day of culture.

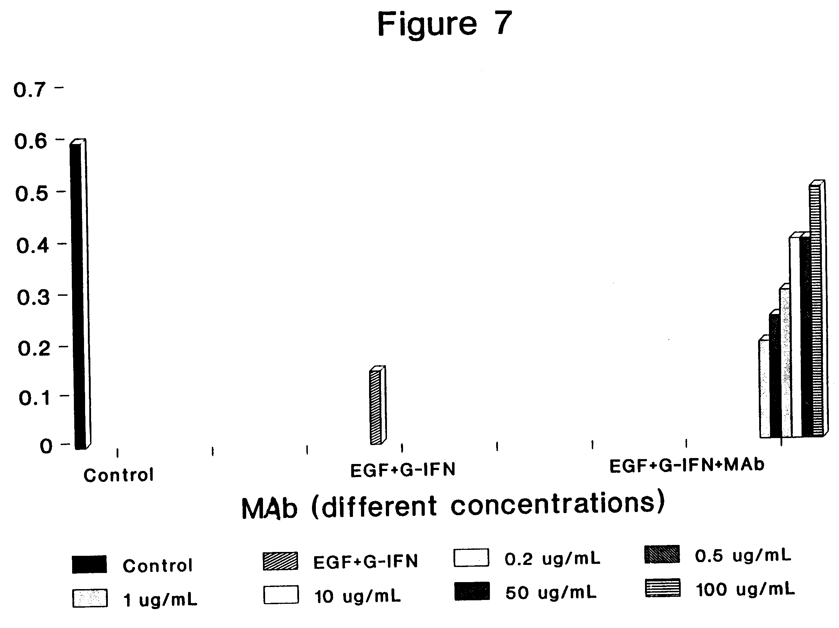

- Figure 7-: reflects the monoclonal antibody's antagonist effect on EGF's biological activity. The cells were cultivated with EGF (1 µg/mL); Gamma-IFN (100 µg/mL); EGF + G-IFN; and different concentrations of monoclonal antibody in combination with Gamma-IFN and EGF. Determination of the proteins in the cellular monolayer was performed the seventh day of culture.

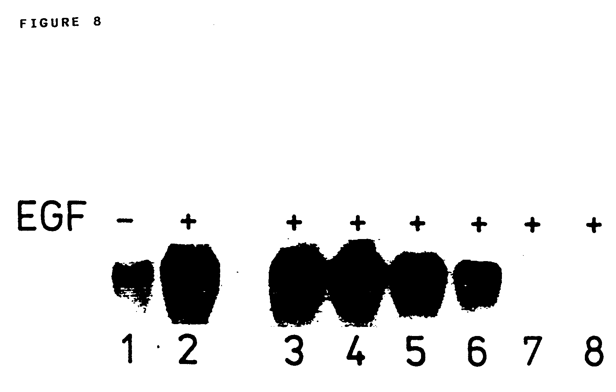

- Figure 8-: shows the inhibition of EGF-stimulated autophosphorylation caused by the monoclonal antibody in placental membrane tissue. The membranes were incubated with ³²P-ATP in absence (band 1) or in the presence of EGF (bands 2-8) and were analyzed via polyacrylamide gel electrophoresis and autoradiography. The effects of increases in the concentration of the MAb is observed in the incorporation of Phosphorus-32 in the EGF-R. Bands 2 and 3 without the MAb, band 4: 1.8 µg/mL, band 5: 6 µg/mL, band 6: 18 µg/mL, band 7: 60 µg/mL and band 8: 180 µg/mL of the MAb.
- Figure 9-: demonstrates that the Fab Fragments retain the same capacity as the monoclonal antibody in the A431 cells to inhibit EGF binding to its receptor. A competitive assay was performed with a microsomal fraction of human placenta and different concentrations of the MAb, and its Fab fragment. EGF labeled with 125-Iodine was used as the radio tracer.
- Figure 10-: shows that the Fab Fragments retain the same capacity as the native monoclonal antibody in the EGF's antagonist effect on A-431 cells. The cells were cultured in different concentrations of the Mab and its fragments. Determination of the proteins in the cellular monolayer was upon the seventh day of culture.

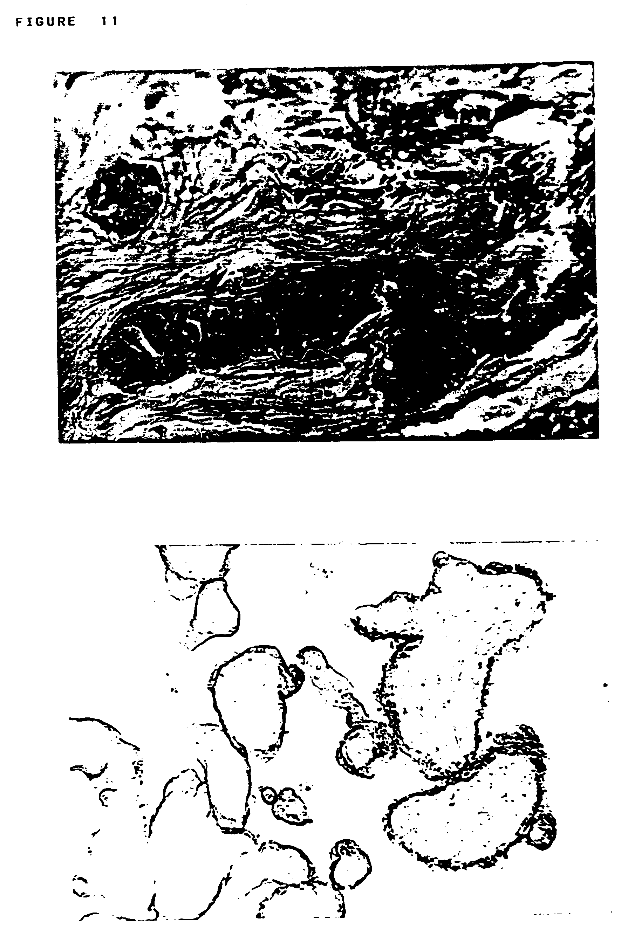

- Figure 11-: reports the results of the immunohistochemical analysis of breast cancer. Epithelial cells of invasive ductal carcinoma are heavily stained.
- Figure 12-: refers to the monoclonal antibody's effect on the H-125 cellular line; accelerated growth of the control group (which doubled its growth several times) can be observed. The group treated with the lowest concentration (10 µg/mL) shows slightly slower growth than the control group. Groups treated with higher concentrations showed some cellular growth during days 1 and 2. Starting on Day 3, the culture entered a stable phase with no growth, by Day 5 there is a 50% inhibition in comparison to the control.
- Figure 13-: shows the monoclonal antibody's effect on the U-1752 cellular line. The groups treated with the lowest concentration (10 micrograms/mL) showed no growth differences in comparison to the control. The groups treated with higher concentrations showed some cellular growth during the first two days starting on Day 3, the culture entered a stable phase during which there was no cellular growth by Day 5, the inhibition was 50% in comparison to the control.
- Figure 14-: shows the cytotoxic cellular effect in the presence of rabbit complement of the monoclonal antibodies in the H-125 cellular line.
- Figure 15-: describes the effect of the combination of the monoclonal antibodies at a fixed concentration with two different concentrations of N-acetyl GM3 in relation to the controls. Control group No. 1 an irrelevant antibody with the same concentration as the monoclonal antibody against EGF Receptor. Control groups No. 2 and No. 3 contains the same irrelevant antibody at the same concentration together with N-acetyl GM3 in two different concentrations. Control group No. 4 contains the monoclonal antibody alone at a concentration of 30 µg/mL. Groups No. 5 and 6 contain monoclonal antibody against EGF Receptor at a concentration of 30 µg/mL and N-acetyl GM3 at concentrations of 2.5 and 5 µg/mL, respectively.
- Tables 1 and 2-: show the monoclonal antibody recognition pattern in normal and tumoral tissue performed in fresh tissue in cryostatic sections.
- Cell line deposits-: A431 = ATCC CRL 1555

**TABLE 1**

| MONOCLONAL ANTIBODY RECOGNITION IN NORMAL ORGANS* | | | | | |
|---|---|---|---|---|---|
| ORGAN | REACTIVITY | | | | |
| | - | -/+ | + | ++ | +++ |
| Heart | | 3/3 | | | |
| Prostate | | | | 2/2 | |
| Liver | | | | 3/3 | |
| Lung | | | | 3/3 | |
| Kidney | | | | 2/2- | |
| Spleen | | | 2/2 | | |
| Ovary | | | | 2/2 | |
| Pancreas | | | | 1/2 | 1/2 |
| Testis | | | | 1/2 | 1/2 |
| Adrenal Glands | | | | 1/1 | |
| Hypophysis | 2/2 | | | | |
| Lymph Nodes | 2/2 | | | | |
| Medulla | 1/1 | | | | |
| Thyroid | | | | 2/2 | |
| Tonsils | | | | | 3/3 |
| Stomach | | | | 2/2 | |
| Small Intestine | | | | 2/2 | |
| Large Intestine | | | | 2/2 | |
| Skin | | | | 2/2 | |
| Submaxillary Gland | | | | 2/2 | |
| Placenta | | | | 1/1 | |
| Brain | | | | 1/1 | |
| Cerebellum | | | | 1/1 | |
| Legend no reaction (-); weak reaction (+); moderate reaction (++); strong reaction (+++). cryostat sections | | | | | |

**TABLE 2**

| MONOCLONAL ANTIBODY RECOGNITION IN TUMORS | | | | |
|---|---|---|---|---|
| Organ | Reactivity | | | |
| | - | -/+ | ++ | +++ |
| Glioma | | | | 1/1 |
| Meningioma | | | | 1/1 |
| Malignant Histiocytoma | | | | 1/1 |
| Neurofibrosarcoma | | | | 1/1 |
| Breast cancer | | 4/14 | 5/14 | 5/14 |
| Lung Cancer | | | 3/3 | |
| Tumors of the Head and Neck | | | | 5/5 |
| Basal Carcinoma | | 1/1 | | |
| Legend no reaction (-); weak reaction (+); moderate reaction (++). | | | | |

## Claims

1. A monoclonal antibody which recognizes human Epidermal Growth Factor (EGF) receptor, is capable of inhibiting EGF binding to the receptor, is capable of inhibiting growth of EGF-dependent tumor cells, and in addition has the following properties:
a) has antibody-dependent cellular cytotoxicity,
b) has complement-mediated cytotoxic activity,
c) is capable of producing a synergetic effect in inhibiting proliferation of tumor cells if combined with N-acetyl GM3 ganglioside, and
d) is capable of producing a synergetic effect in inhibiting proliferation of tumor cells if combined with a monoclonal antibody against EGF,
or a derivative of said monoclonal antibody selected from humanized, chimeric, reshaped, bispecific, conjugated and anti-idiotypic forms of it,
or a fragment of said monoclonal antibody or said derivative selected from Fv, Fab, Fab' and F(ab')₂ fragments of it.

2. A monoclonal antibody according to claim 1, which recognizes both human EGF receptor present in normal cells and EGF receptor present in tumoral cells.

3. A monoclonal antibody according to claim 2, which recognizes both human placental EGF receptor and EGF receptor from the A-431 tumor cell line (ATCC CRL 1555).

4. A monoclonal antibody according to any one of claims 1 to 3, which is capable of inhibiting the growth of the EGF-dependent lung tumor cell lines U-1752 and/or H-125.

5. A monoclonal antibody according to any one of claims 1 to 4, which has an antibody-dependent cellular cytotoxicity in a test using the A-431 cell line (ATCC CRL 1555) as target cells.

6. A monoclonal antibody according to any one of claims 1 to 5, which has a complement-mediated cytotoxic activity in a test using the A-431 cell line (ATCC CRL 1555) as target cells.

7. A monoclonal antibody according to any one of claims 1 to 6, which is capable of producing a synergetic effect in inhibiting proliferation of H-125 tumor cells if combined with the ganglioside N-acetyl GM3.

8. A monoclonal antibody according to any one of claims 1 to 7, which is capable of producing a synergetic effect in inhibiting proliferation of H-125 tumor cells if combined with an anti-EGF monoclonal antibody.

9. A monoclonal antibody according to any one of claims 1 to 8, which is an immunoglobuline of the IgG 2a-type.

10. A composition comprising a monoclonal antibody according to any one of claims 1 to 9, together with a carrier, a diluent or an excipient therefor.

11. A pharmaceutical composition for use in a therapeutical or prophylactic treatment of malignant neoplasms or pre-neoplastic diseases, comprising a therapeutically or prophylactically effective amount of a monoclonal antibody according to any one of claims 1 to 9, together with a carrier, a diluent or an excipient therefor.

12. A pharmaceutical composition according to claim 11, which contains N-acetyl GM3 ganglioside.

13. A pharmaceutical composition according to claim 11 or claim 12, which contains a monoclonal antibody against EGF.

14. A process for preparing a monoclonal antibody which recognizes human Epidermal Growth Factor (EGF) receptor, comprising the steps of immunizing a test animal with human EGF receptor or an antigenic derivative or fragment thereof, isolating antibody-producing cells from the immunized animal, immortalizing said antibody-producing cells, selecting from said immortalized cells a cell which produces a monoclonal antibody which recognizes human EGF receptor, is capable of inhibiting EGF binding to the receptor, is capable of inhibiting growth of EGF-dependent tumor cells, and in addition has the following properties:
a) has antibody-dependent cellular cytotoxicity,
b) has complement-mediated cytotoxic activity,
c) is capable of producing a synergetic effect in inhibiting proliferation of tumor cells if combined with N-acetyl GM3 ganglioside, and
d) is capable of producing a synergetic effect in inhibiting proliferation of tumor cells if combined with a monoclonal antibody against EGF,
and isolating monoclonal antibody produced by said selected cells.

15. A process according to claim 14, wherein the step of immortalizing the antibody-producing cells isolated from the immunized animal is carried out by fusing the cells with myeloma cells to produce hybridoma cells.

16. A process according to claim 14 or claim 15, wherein the test animal used in the immunisation step is a rodent animal, preferably a mouse or rat.

17. A process according to any one of claims 14 to 16, wherein the test animal is immunized with human placenta EGF receptor.

18. A process according to any one of claims 14 to 17, wherein the selected monoclonal antibody recognizes both human EGF receptor present in normal cells and EGF receptor present in tumoral cells.

19. A process according to any one of claims 14 to 18, wherein the selected monoclonal antibody recognizes both human placental EGF receptor and EGF receptor from the A-431 tumor cell line (ATCC CRL 1555).

20. A process according to any one of claims 14 to 19, wherein the selected monoclonal antibody is capable of inhibiting the growth of the EGF-dependent lung tumor cell lines U-1752 and/or H-125.

21. A process according to any one of claims 14 to 20, wherein the selected monoclonal antibody has an antibody-dependent cellular cytotoxicity in a test using the A-431 cell line (ATCC CRL 1555) as target cells.

22. A process according to any one of claims 14 to 21, wherein the selected monoclonal antibody has a complement-mediated cytotoxic activity in a test using the A-431 cell line (ATCC CRL 1555) as target cells.

23. A process according to any one of claims 14 to 22, wherein the selected monoclonal antibody is capable of producing a synergetic effect in inhibiting proliferation of H-125 tumor cells if combined with the ganglioside N-acetyl GM3.

24. A process according to any one of claims 14 to 23, wherein the selected monoclonal antibody is capable of producing a synergetic effect in inhibiting proliferation of H-125 tumor cells if combined with a anti-EGF monoclonal antibody.

25. A process according to any one of claims 14 to 24, wherein the selected monoclonal antibody is an immunoglobuline of the IgG 2a-type.

26. A process according to any one of claims 14 to 25, wherein immortalized cells producing the selected monoclonal antibody are cultivated, either in vitro in a culture medium of the proper tissue, or in vivo in a histocompatible body fluid, or in fermenters such as production medium, followed by separation of the immortalized cells from said medium.

27. A process according to any one of claims 14 to 26, wherein the monoclonal antibody produced is purified and optionally derivatized or fragmented.

28. An immortalized cell producing a monoclonal antibody according to any one of claim 1 to 9.

29. Use of a monoclonal antibody according to any one of claims 1 to 9 for preparing a pharmaceutical composition for therapeutical or prophylactic treatment of malignant neoplasms or pre-neoplastic diseases.

## Patentansprüche

1. Monoklonaler Antikörper, der den Human-Epidermiswachstumsfaktor-(EGF)-Rezeptor erkennt, die Bindung von EGF an den Rezeptor zu hemmen vermag, das Wachstum von EGF-abhängigen Tumorzellen zu hemmen vermag und außerdem die folgenden Eigenschaften hat:
a) eine antikörperabhängige zelluläre Cytotoxizität;
b) eine komplementvermittelte cytotoxische Aktivität;
c) die Fähigkeit zur Erzeugung einer synergistischen Wirkung bei der Hemmung der Proliferation von Tumorzellen, wenn er mit dem Gangliosid N-Acetyl-GM3 kombiniert ist; und
d) die Fähigkeit zur Erzeugung einer synergistischen Wirkung bei der Hemmung der Proliferation von Tumorzellen, wenn er mit einem monoklonalen Antikörper gegen EGF kombiniert ist;
oder ein Derivat des monoklonalen Antikörpers, das aus humanisierten, chimärischen, umgeformten, bispezifischen, konjugierten und antiidiotypischen Formen desselben ausgewählt ist;
oder ein Fragment des monoklonalen Antikörpers oder des Derivats, das aus Fv-, Fab-, Fab'- und F(ab')₂-Fragmenten derselben ausgewählt ist.

2. Monoklonaler Antikörper gemäß Anspruch 1, der sowohl Human-EGF-Rezeptor, der in normalen Zellen vorhanden ist, als auch EGF-Rezeptor, der in Tumorzellen vorhanden ist, erkennt.

3. Monoklonaler Antikörper gemäß Anspruch 2, der sowohl Human-Plazenta-EGF-Rezeptor als auch EGF-Rezeptor aus der Tumorzellinie A-431 (ATCC CRL 1555) erkennt.

4. Monoklonaler Antikörper gemäß einem der Ansprüche 1 bis 3, der das Wachstum der EGF-abhängigen Lungentumorzellinien U-1752 und/oder H-125 zu hemmen vermag.

5. Monoklonaler Antikörper gemäß einem der Ansprüche 1 bis 4, der in einem Test unter Verwendung der Zellinie A-431 (ATCC CRL 1555) als Targetzellen eine antikörperabhängige zelluläre Cytotoxizität aufweist.

6. Monoklonaler Antikörper gemäß einem der Ansprüche 1 bis 5, der in einem Test unter Verwendung der Zellinie A-431 (ATCC CRL 1555) als Targetzellen eine komplementvermittelte cytotoxische Aktivität aufweist.

7. Monoklonaler Antikörper gemäß einem der Ansprüche 1 bis 6, der eine synergistische Wirkung bei der Hemmung der Proliferation von H-125-Tumorzellen zu erzeugen vermag, wenn er mit dem Gangliosid N-Acetyl-GM3 kombiniert ist.

8. Monoklonaler Antikörper gemäß einem der Ansprüche 1 bis 7, der eine synergistische Wirkung bei der Hemmung der Proliferation von H-125-Tumorzellen zu erzeugen vermag, wenn er mit einem monoklonalen Anti-EGF-Antikörper kombiniert ist.

9. Monoklonaler Antikörper gemäß einem der Ansprüche 1 bis 8, bei dem es sich um ein Immunglobulin des Typs IgG 2a handelt.

10. Zusammensetzung, die einen monoklonalen Antikörper gemäß einem der Ansprüche 1 bis 9 zusammen mit einem Träger, einem Verdünnungsmittel oder einem Arzneimittelhilfsstoff für den Antikörper umfaßt.

11. Pharmazeutische Zusammensetzung zur Verwendung bei einer therapeutischen oder prophylaktischen Behandlung maligner Neoplasmen oder präneoplastischer Erkrankungen, umfassend eine therapeutisch oder prophylaktisch wirksame Menge eines monoklonalen Antikörpers gemäß einem der Ansprüche 1 bis 9 zusammen mit einem Träger, einem Verdünnungsmittel oder einem Arzneimittelhilfsstoff für den Antikörper.

12. Pharmazeutische Zusammensetzung gemäß Anspruch 11, die das Gangliosid N-Acetyl-GM3 enthält.

13. Pharmazeutische Zusammensetzung gemäß Anspruch 11 oder 12, die einen monoklonalen Antikörper gegen EGF enthält.

14. Verfahren zur Herstellung eines monoklonalen Antikörpers, der den Human-Epidermiswachstumsfaktor-(EGF)-Rezeptor erkennt, umfassend die Schritte des Immunisierens eines Versuchstiers mit Human-EGF-Rezeptor oder einem antigenen Derivat oder Fragment davon, des Isolierens antikörpererzeugender Zellen aus dem immunisierten Tier, des Immortalisierens der antikörpererzeugenden Zellen, des Auswählens einer Zelle aus den immortalisierten Zellen, die einen monoklonalen Antikörper erzeugt, der Human-EGF-Rezeptor erkennt, die Bindung von EGF an den Rezeptor zu hemmen vermag, das Wachstum von EGF-abhängigen Tumorzellen zu hemmen vermag und außerdem die folgenden Eigenschaften hat:
a) eine antikörperabhängige zelluläre Cytotoxizität;
b) eine komplementvermittelte cytotoxische Aktivität;
c) die Fähigkeit zur Erzeugung einer synergistischen Wirkung bei der Hemmung der Proliferation von Tumorzellen, wenn er mit dem Gangliosid N-Acetyl-GM3 kombiniert ist; und
d) die Fähigkeit zur Erzeugung einer synergistischen Wirkung bei der Hemmung der Proliferation von Tumorzellen, wenn er mit einem monoklonalen Antikörper gegen EGF kombiniert ist;
und des Isolierens des von den ausgewählten Zellen erzeugten monoklonalen Antikörpers.

15. Verfahren gemäß Anspruch 14, wobei der Schritt des Immortalisierens der aus dem immunisierten Tier isolierten antikörpererzeugenden Zellen durchgeführt wird, indem man die Zellen unter Bildung von Hybridomzellen mit Myelomzellen fusioniert.

16. Verfahren gemäß Anspruch 14 oder 15, wobei das im Immunisierungsschritt verwendete Versuchstier ein Nagetier ist, vorzugsweise eine Maus oder eine Ratte.

17. Verfahren gemäß einem der Ansprüche 14 bis 16, wobei das Versuchstier mit Human-Plazenta-EGF-Rezeptor immunisiert wird.

18. Verfahren gemäß einem der Ansprüche 14 bis 17, wobei der ausgewählte monoklonale Antikörper sowohl Human-EGF-Rezeptor, der in normalen Zellen vorhanden ist, als auch EGF-Rezeptor, der in Tumorzellen vorhanden ist, erkennt.

19. Verfahren gemäß einem der Ansprüche 14 bis 18, wobei der ausgewählte monoklonale Antikörper sowohl Human-Plazenta-EGF-Rezeptor als auch EGF-Rezeptor aus der Tumorzellinie A-431 (ATCC CRL 1555) erkennt.

20. Verfahren gemäß einem der Ansprüche 14 bis 19, wobei der ausgewählte monoklonale Antikörper das Wachstum der EGF-abhängigen Lungentumorzellinien U-1752 und/oder H-125 zu hemmen vermag.

21. Verfahren gemäß einem der Ansprüche 14 bis 20, wobei der ausgewählte monoklonale Antikörper in einem Test unter Verwendung der Zellinie A-431 (ATCC CRL 1555) als Targetzellen eine antikörperabhängige zelluläre Cytotoxizität aufweist.

22. Verfahren gemäß einem der Ansprüche 14 bis 21, wobei der ausgewählte monoklonale Antikörper in einem Test unter Verwendung der Zellinie A-431 (ATCC CRL 1555) als Targetzellen eine komplementvermittelte cytotoxische Aktivität aufweist.

23. Verfahren gemäß einem der Ansprüche 14 bis 22, wobei der ausgewählte monoklonale Antikörper eine synergistische Wirkung bei der Hemmung der Proliferation von H-125-Tumorzellen zu erzeugen vermag, wenn er mit dem Gangliosid N-Acetyl-GM3 kombiniert ist.

24. Verfahren gemäß einem der Ansprüche 14 bis 23, wobei der ausgewählte monoklonale Antikörper eine synergistische Wirkung bei der Hemmung der Proliferation von H-125-Tumorzellen zu erzeugen vermag, wenn er mit einem monoklonalen Anti-EGF-Antikörper kombiniert ist.

25. Verfahren gemäß einem der Ansprüche 14 bis 24, wobei es sich bei dem ausgewählten monoklonalen Antikörper um ein Immunglobulin des Typs IgG 2a handelt.

26. Verfahren gemäß einem der Ansprüche 14 bis 25, wobei immortalisierte Zellen, die den ausgewählten monoklonalen Antikörper erzeugen, entweder in vitro in einem Kulturmedium des entsprechenden Gewebes oder in vivo in einer histokompatiblen Körperflüssigkeit oder in Fermentern, wie Produktionsmedium, kultiviert werden und die immortalisierten Zellen anschließend von dem Medium abgetrennt werden.

27. Verfahren gemäß einem der Ansprüche 14 bis 26, wobei der erzeugte monoklonale Antikörper gereinigt und gegebenenfalls derivatisiert oder fragmentiert wird.

28. Immortalisierte Zelle, die einen monoklonalen Antikörper gemäß einem der Ansprüche 1 bis 9 erzeugt.

29. Verwendung eines monoklonalen Antikörpers gemäß einem der Ansprüche 1 bis 9 zur Herstellung einer pharmazeutischen Zusammensetzung für die therapeutische oder prophylaktische Behandlung maligner Neoplasmen oder präneoplastischer Erkrankungen.

## Revendications

1. Anticorps monoclonal qui reconnaît le récepteur du facteur de croissance épidermique EGF (Epidermal Growth Factor) humain, qui est capable d'inhiber la liaison de l'EGF au récepteur, qui est capable d'inhiber la croissance des cellules tumorales dépendantes de l'EGF, et qui présente en plus les propriétés suivantes :
a) il possède une cytotoxicité cellulaire dépendante des anticorps,
b) il possède une activité cytotoxique à médiation par le complément,
c) il est capable de produire un effet de synergie lors de l'inhibition de la prolifération des cellules tumorales s'il est associé au ganglioside N-acétyl-GM3, et
d) il est capable de produire un effet de synergie lors de l'inhibition de la prolifération des cellules tumorales s'il est associé à un anticorps monoclonal dirigé contre l'EGF,
ou dérivé dudit anticorps monoclonal choisi parmi les formes humanisées, chimériques, reformées, bispécifiques, conjuguées et anti-idiotypiques de celui-ci,
ou bien fragment dudit anticorps monoclonal ou dudit dérivé choisi parmi les fragments Fv, Fab, Fab' et F(ab')₂ de celui-ci.

2. Anticorps monoclonal selon la revendication 1, qui reconnaît à la fois le récepteur de l'EGF humain présent dans les cellules normales et le récepteur de l'EGF présent dans les cellules tumorales.

3. Anticorps monoclonal selon la revendication 2, qui reconnaît à la fois le récepteur de l'EGF placentaire humain et le récepteur de l'EGF provenant de la lignée de cellules tumorales A-431 (ATCC CRL 1555).

4. Anticorps monoclonal selon l'une quelconque des revendications 1 à 3, qui est capable d'inhiber la croissance dépendante de l'EGF des lignées de cellules tumorales pulmonaires U-1752 et/ou H-125.

5. Anticorps monoclonal selon l'une quelconque des revendications 1 à 4, qui possède une cytotoxicité cellulaire dépendante des anticorps dans un test utilisant la lignée cellulaire A-431 (ATCC CRL 1555) comme cellules cibles.

6. Anticorps monoclonal selon l'une quelconque des revendications 1 à 5, qui possède une cytotoxicité cellulaire à médistion par le complément dans un test utilisant la lignée cellulaire A-431 (ATCC CRL 1555) comme cellules cibles.

7. Anticorps monoclonal selon l'une quelconque des revendications 1 à 6, qui est capable de produire un effet de synergie lors de l'inhibition de la prolifération des cellules tumorales H-125 s'il est associé au ganglioside N-acétyl-GM3.

8. Anticorps monoclonal selon l'une quelconque des revendications 1 à 7, qui est capable de produire un effet de synergie lors de l'inhibition de la prolifération des cellules tumorales H-125 s'il est associé à un anticorps monoclonal anti-EGF.

9. Anticorps monoclonal selon l'une quelconque des revendications 1 à 8, qui est une immunoglobuline du type IgG 2a.

10. Composition contenant un anticorps monoclonal selon l'une quelconque des revendications 1 à 9, ensemble avec un véhicule, un diluant ou un excipient pour celle-ci.

11. Composition pharmaceutique pour utilisation dans un traitement thérapeutique ou prophylactique de néoplasmes malins ou de maladies pré-néoplasiques, comprenant une quantité thérapeutiquement ou prophylactiquement efficace d'un anticorps monoclonal selon l'une quelconque des revendications 1 à 9, ensemble avec un véhicule, un diluant ou un excipient pour celle-ci.

12. Composition pharmaceutique selon la revendication 11, comprenant du ganglioside N-acétyl-GM3.

13. Composition pharmaceutique selon la revendication 11 ou la revendication 12, qui contient un anticorps monoclonal dirigé contre l'EGF.

14. Procédé de préparation d'un anticorps monoclonal qui reconnaît le récepteur du facteur de croissance épidermique EGF (Epidermal Growth Factor), comprenant les étapes consistant à immuniser un animal de test avec un récepteur de l'EGF humain ou avec un dérivé ou fragment antigénique de celui-ci ; isoler les cellules produisant des anticorps à partir de l'animal immunisé ; immortaliser lesdites cellules produisant des anticorps ; sélectionner parmi lesdites cellules immortalisées une cellule qui produit un anticorps monoclonal qui reconnaît le récepteur de l'EGF humain, qui est capable d'inhiber la liaison de l'EGF au récepteur, qui est capable d'inhiber la croissance des cellules tumorales dépendantes de l'EGF, et qui présente en plus les propriétés suivantes :
a) il possède une cytotoxicité cellulaire dépendante des anticorps,
b) il possède une activité cytotoxique a médiation par le complément,
c) il est capable de produire un effet de synergie lors de l'inhibition de la prolifération des cellules tumorales s'il est associé au ganglioside N-acétyl-GM3, et
d) il est capable de produire un effet de synergie lors de l'inhibition de la prolifération des cellules tumorales s'il est associé a un anticorps monoclonal dirigé contre l'EGF ;
et isoler l'anticorps monoclonal produit par lesdites cellules sélectionnées.

15. Procédé selon la revendication 14, dans lequel l'étape d'immortalisation des cellules produisant des anticorps isolées à partir de l'animal immunisé se fait en fusionnant les cellules avec des cellules de myélome afin de produire des cellules d'hybridome.

16. Procédé selon la revendication 14 ou la revendication 15, dans lequel l'animal de test utilisé dans l'étape d'immunisation est un animal rongeur, de préférence une souris ou un rat.

17. Procédé selon l'une quelconque des revendications 14 à 16, dans lequel l'animal de test est immunisé avec le récepteur de l'EGF placentaire humain.

18. Procédé selon l'une quelconque des revendications 14 à 17, dans lequel l'anticorps monoclonal sélectionné reconnaît à la fois le récepteur de l'EGF humain présent dans les cellules normales et le récepteur de l'EGF présent dans les cellules tumorales.

19. Procédé selon l'une quelconque des revendications 14 à 18, dans lequel l'anticorps monoclonal sélectionné reconnaît à la fois le récepteur de l'EGF placentaire humain et le récepteur de l'EGF provenant de la lignée de cellules tumorales A-431 (ATCC CRL 1555).

20. Procédé selon l'une quelconque des revendications 14 à 19, dans lequel l'anticorps monoclonal sélectionné est capable d'inhiber la croissance dépendante de l'EGF des lignées de cellules tumorales pulmonaires U-1752 et/ou H-125.

21. Procédé selon l'une quelconque des revendications 14 à 20, dans lequel l'anticorps monoclonal sélectionné possède une cytotoxicité cellulaire dépendante des anticorps dans un test utilisant la lignée cellulaire A-431 (ATCC CRL 1555) comme cellules cibles.

22. Procédé selon l'une quelconque des revendications 14 à 21, dans lequel l'anticorps monoclonal sélectionné possède une cytotoxicité cellulaire à médiation par le complément dans un test utilisant la lignée cellulaire A-431 (ATCC CRL 1555) comme cellules cibles.

23. Procédé selon l'une quelconque des revendications 14 à 22, dans lequel l'anticorps monoclonal sélectionné est capable de produire un effet de synergie lors de l'inhibition de la prolifération des cellules tumorales H-125 s'il est associé au ganglioside N-acétyl-GM3.

24. Procédé selon l'une quelconque des revendications 14 à 23, dans lequel l'anticorps monoclonal sélectionné est capable de produire un effet de synergie lors de l'inhibition de la prolifération des cellules tumorales H-125 s'il est associé à un anticorps monoclonal anti-EGF.

25. Procédé selon l'une quelconque des revendications 14 à 24, dans lequel l'anticorps monoclonal sélectionné est une immunoglobuline du type IgG 2a.

26. Procédé selon l'une quelconque des revendications 14 à 25, dans lequel les cellules immortalisées produisant l'anticorps monoclonal sélectionné sont cultivées in vitro dans un milieu de culture du tissu approprié, ou bien in vivo dans un fluide corporel histocompatible, ou dans des fermenteurs tels que le milieu de production, suivi de la séparation des cellules immortalisées d'avec ledit milieu.

27. Procédé selon l'une quelconque des revendications 14 à 26, dans lequel l'anticorps monoclonal produit est purifié et facultativement dérivatisé ou fragmenté.

28. Cellule immortalisée produisant un anticorps monoclonal selon l'une quelconque des revendications 1 à 9.

29. Utilisation d'un anticorps monoclonal selon l'une quelconque des revendications 1 à 9 pour préparer une composition pharmaceutique pour le traitement thérapeutique ou prophylactique de néoplasmes malins ou de maladies pré-néoplasiques.
